# EUROPÄISCHE PATENTSCHRIFT

(11) **EP 0 596 212 B1**
(45) Veröffentlichungstag und Bekanntmachung des Hinweises auf die Patenterteilung: **03.12.1997**
(21) Anmeldenummer: 93114014.9
(22) Anmeldetag: 02.09.1993
(51) Int. Cl.: B65D 75/58, A61L 9/12

(54) **Behälter zur Aufnahme von aromatischen Substanzen**
Receptacle for aromatic substances
Récipient pour substances aromatiques

(30) Priorität: 31.10.1992 DE 4236886
(43) Veröffentlichungstag der Anmeldung: 11.05.1994
(73) Patentinhaber: KLOCKE VERPACKUNGS-SERVICE GmbH, D-76356 Weingarten (DE); MANKA, F-68000 Colmar (FR)
(72) Erfinder: Huber, Hans-Peter, D-76684 Östringen (DE); Klocke, Hartmut, D-76133 Karlsruhe (DE); Wendel, Herbert, F-57200 Sarre-Guemines (FR)
(74) Vertreter: Sartorius, Peter, Dipl.-Ing.

(56) Entgegenhaltungen:
- DE-C- 3 490 012
- US-A- 4 145 001
- US-A- 4 161 283

## Beschreibung

Die Erfindung bezieht sich auf einen Behälter zur Aufnahme von aromatischen Substanzen, der mittels einer aus mehreren Schichten gebildeten Verbundfolie aromadicht verschließbar ist, wobei die Verbundfolie auf einer Behälteroberseite aus einer aromadichten, mindestens zwei Schichten aufweisenden Außenfolie und einer darunterliegenden, aromadurchlässigen, aus zwei Schichten bestehenden Innenfolie gebildet ist, wobei die aromadichte Außenfolie mit der darunterliegenden, aus einem Polymerwerkstoff gebildeten, permeablen Zwischenschicht über einen Kleber verbunden ist.

Aus der DE-A-31 49 508 ist es bekannt, einen Duftbeutel mit zwei an den Rändern untereinander verbundenen und einen Duftstoff umschließenden, aromadichten Folienflächen zu verschließen, wobei die Folienfläche auf der Beutelseite aus einer Doppelschicht zusammengesetzt ist, die aus einer aromadurchlässigen Innenschicht und einer aromadichten Außenschicht besteht. Die Ränder können versiegelt oder verschweißt sein.

Es ist ferner ein Behälter zur Aufnahme von Duftstoffen aus der DE-C-34 90 012 bekannt, der eine Kammer mit einer Wand aus polymerem Material aufweist, die nach Abreißen einer Deckfolie das Diffundieren der Dämpfe der aktiven Substanz in die Umgebung erlaubt. Die Oberfläche der unteren, polymeren, dampfdurchlässigen Wand ist von einer Papierschicht mit einer Dicke zwischen 20 und 100 µm und einem Gewicht zwischen 20 und 100 g/m² bedeckt. An der Papierschicht haftet eine zweite polymere, mit Al kaschierte Wand. Der Anwender kann zum Freisetzen der Duftstoffe die aus Al und polymerem Werkstoff gebildete Schutzschicht aufreißen, wobei das Papier in der Mitte gespalten wird. Die zwischen den beiden polymeren Schichten vorgesehene Papierschicht hat den wesentlichen Nachteil, daß bereits vor dem Abreißen der oberen Deckfolie während einer längeren Lagerzeit die Papierschicht mit Duftstoff getränkt wird, so daß dieser über die Papierschicht an der Nahtstelle zwischen den beiden aus polymerem Werkstoff gebildeten Schichten nach außen diffundiert und schon vor Ingebrauchnahme ein großer Anteil der Duftstoffe verlorengeht. Daraus ergibt sich eine sehr geringe Lagerstabilität des verschlossenen Beutels.

Die US-A-4 145 001 zeigt einen Behälter, insbesondere in Figur 3 und 5, mit einer Schicht, die aus PE gebildet ist, und einer zweiten inneren Schicht, die aus einem Material besteht, das einen guten Wärmeverbund mit der durchlässigen Schicht bildet, beispielsweise wenn LDPE oder Äthylenvinylacetat-Copolymer als durchlässige Schicht eingesetzt werden soll. Die zweite Schicht kann beispielsweise aus Äthylenacrylsäure-Copolymer gebildet sein, die einen guten Wärmeverbund darstellt. Wie aus Spalte 5, Zeile 35 bis 40 der US-Anmeldung hervorgeht, ist es wichtig, daß eine einwandfreie Funktion des Behälters nur dann gewährleistet ist, wenn die Verbindung zwischen der zweiten inneren Schicht und der äußeren Schicht, die aus Al gebildet ist, größer ist als der Wärmeverbund, der zwischen der abnehmbaren und der durchlässigen Schicht herrscht. Unter Berücksichtigung vorstehender Ausführungen besteht der wichtigste Unterschied zu der bekannten Behälterausbildung darin, daß die Klebeverbindung bzw. Klebeschicht zwischen der Barriereschicht, dem äußeren Laminat und dem Polypropylen eine geringere Haftfähigkeit aufweist als die Verbindung zwischen dem Polypropylen und der Klebeverbindung zwischen dem Polypropylen und dem Polyäthylen. Das bedeutet, daß die Propylenschicht stets auf der verbleibenden Schicht der erfindungsgemäßen Anordnung bleibt, wenn die beiden Schichten bzw. Laminate abgezogen werden. Durch die Verwendung des auf der unteren Schicht haftenden Polypropylens wird eine optimale Abdichtung auf dem Behälter gewährleistet, wobei das äußere Laminat lediglich aus zwei Schichten besteht, die aromadicht ausgebildet sind, da bei Verwendung eines dieser Materialien im Eckbereich keine Substanzen aufgesaugt werden und damit entweichen können.

Demgemäß besteht die Erfindungsaufgabe darin, eine Verbundfolie für einen Behälter zur Aufnahme von aromatischen Substanzen zu schaffen, die verträglich gegenüber dem Füllgut ist, eine einwandfreie Lagerstabilität des Füllguts sichert und beim Entfernen der Folie eine einwandfreie Trennung zwischen der impermeablen und permeablen Folie sicherstellt, ohne daß dabei Füllstoffe bzw. Aromastoffe beim Trennvorgang von der Oberfläche der verbleibenden Folie abspritzen.

Gelöst wird die Aufgabe erfindungsgemäß dadurch, daß der Kleber eine geringere Haftfähigkeit zu der Zwischenschicht aufweist als der zweite Kleber zwischen der Zwischenschicht und der nachfolgenden vierten, mit dem Behälter verbundenen Schicht, die aus Polyäthylen besteht, und daß mit Bezug auf den Gesamtverbund der Folie die dritte bzw. die zur Innenfolie gehörende Zwischenschicht aus einem geschäumten Polypropylen gebildet ist.

Hierzu ist es vorteilhaft, daß die Dichte der dritten bzw. oberen Schicht so gewählt ist, daß die aus den beiden Schichten gebildete Innenfolie oder Membranfolie beim Abziehen der Außenfolie auf dem Behälter haften bleibt, und daß die dritte Schicht bzw. das geschäumte Polypropylen im Randbereich fest mit dem darunterliegenden Polyäthylen verbunden ist und das geschäumte Polypropylen und/oder die darunterliegenden Schichten im Randbereich der festen Verbindung verdichtet sind. Durch die aus den zahlreichen Schichten gebildete Verbundfolie erhält man eine aromadichte Verbundschicht mit besten Dichteigenschaften, so daß hierdurch die Lagerstabilität gegenüber den bekannten Verbundschichten wesentlich verbessert werden kann. Der verdichtete Randbereich einer oder mehrerer Schichten verhindert ein Diffundieren durch die sonst aromadurchlässigen Schichten unterhalb der Barriereschicht, solange die Außenfolie nicht abgezogen ist.

In weiterer Ausgestaltung der Erfindung ist es vorteilhaft, daß das geschäumte Polypropylen im Bereich der festen Verbindung durch Wärmebehandlung verdichtet ist.

Gemäß einer bevorzugten Ausführungsform der erfindungsgemäßen Lösung ist schließlich vorgesehen, daß das geschäumte Polypropylen über den Kleber ganzflächig mit dem darunterliegenden Polyäthylen fest verbunden und nur im Randbereich und/oder insbesondere im Bereich der Klebe- oder Siegelstelle verdichtet ist.

Von besonderer Bedeutung ist für die vorliegende Erfindung, daß die Außenfolie aus zwei fest miteinander verbundenen Schichten gebildet ist, wobei die eine Schicht als aromadichte Barriereschicht und die andere Schicht aus einem biegsamen bzw. elastischen Material, insbesondere Polyamidwerkstoff, gebildet ist. Durch die Verwendung des geschäumten Polypropylens erhält man zusätzlich eine optimale Abdichtung des Behälters, wenn die aus zwei Schichten gebildete Außenfolie mit der aromadurchlässigen Innenfolie verbunden und auf den Behälter aufgebracht ist, weil dieser Werkstoff, speziell im Randbereich, die aromatische Substanz nicht aufsaugt, insbesondere dann nicht, wenn dieser verdichtet ist. Hierdurch wird also der aromadichte Verschluß noch verbessert und sichergestellt, daß die aromatische Substanz nicht durch die einzelnen Schichten unterhalb der Barriereschicht diffundieren kann. Die oberste, aus einem biegsamen bzw. elastischen Material gebildete Schicht stellt sicher, daß beim Abreißen die Barriereschicht nicht beschädigt wird.

Im Zusammenhang mit der erfindungsgemäßen Ausbildung und Anordnung ist es von Vorteil, daß die Barriereschicht aus einem Metallwerkstoff bzw. aus Al-Material gebildet ist.

Vorteilhaft ist es ferner, daß die aromadichte Barriereschicht mit der anliegenden, aus Polyamid gebildeten Schicht ganzflächig fest verbunden ist.

Ferner ist es vorteilhaft, daß der Behälter zur Aufnahme von aromatischen Substanzen aus zwei ganzflächig miteinander verbundenen Schichten gebildet ist, wobei die Schicht des Behälters aus Polyäthylen und die außenliegende Schicht aus Polyester oder Polypropylen gebildet ist, und daß die einzelnen Schichten über je einen Kaschierkleber ganzflächig fest miteinander verbunden sind. Hierdurch wird eine einwandfreie, hoch beanspruchbare Verbindung zwischen den einzelnen Schichten geschaffen.

Außerdem ist es vorteilhaft, daß die Außenfolie mit der Innenfolie bzw. Membranfolie im Randbereich verschweißt oder über den Kaschierkleber verbunden ist. Hierdurch kann die Barriereschicht im Bereich der Klebestelle von der darunterliegenden, mit dem Behälter verbundenen Schicht abgezogen werden, so daß dadurch über die nicht verdichteten Teile Duftstoffe nach außen diffundieren können.

Eine zusätzliche Möglichkeit ist gemäß einer Weiterbildung der erfindungsgemäßen Verbundfolie, daß die Außenfolie mit der Innenfolie bzw. Membranfolie ganzflächig verschweißt oder über den Kaschierkleber verbunden ist, wobei im Randbereich eine Trennstelle für eine Öffnungslasche vorgesehen ist, die derart ausgebildet ist, daß mit ihr die Außenfolie von der Innenfolie abgezogen und der Verbund zwischen Innenfolie und Außenfolie im Bereich des Kaschierklebers ganzflächig von der Polypropylen-Schicht abgehoben werden kann. Durch die vorteilhafte Anordnung der Öffnungslasche läßt sich die erste, als Stützfolie ausgebildete Schicht mit der Barriereschicht abziehen, ohne diese zu beschädigen. Da die Barriereschicht eine wesentlich höhere Dichte aufweist als das Polypropylenmaterial, bleibt dieses vollständig an der unteren, als Membrane ausgebildeten Schicht haften.

In weiterer Ausgestaltung der Erfindung ist es vorteilhaft, daß der Randbereich der Unterseite der Außenfolie oder der zweiten Schicht mit einer Schutzschicht, insbesondere einem Lackstreifen, zur Verhinderung einer Verbindung der Unterseite der zweiten Schicht mit der Oberseite der dritten Schicht, versehen ist und daß die zweite Schicht der Außenfolie aus einem metallfreien Werkstoff, insbesondere hydrolisiertem Vinylazetat- und/oder Äthylen- und Copolymer-Werkstoff (EVOH und EVA), besteht und mit der ersten Schicht verbunden ist, die aus einem biegsamen Material, insbesondere aus Polyamid oder Polypropylen, besteht. Auf diese Weise erhält man einen recycelbaren Werkstoff.

Wie zuvor beschrieben ist es vorteilhaft, daß die einzelnen Schichten über je einen Zweikomponentenkleber auf PU-Basis, einen Acrylkleber oder ein Koextrudierverfahren ganzflächig miteinander verbunden sind.

Eine zusätzliche Möglichkeit gemäß einer Weiterbildung des erfindungsgemäßen Behälters besteht darin, daß durch Wärmebehandlung des Polypropylenmaterials bzw. der Membranfolie bzw. der Polyäthylenfolie oder durch Eingabe der Substanz in den Behälter und Kontaktaufnahme mit der Schicht die beiden Folien derart transparent gemacht werden, daß die im Behälter aufgenommene Substanz durch die beiden Schichten sichtbar ist, wenn die Außenfolie abgezogen wurde. Das Sichtbarmachen der Substanz kann auch durch die Eingabe der Substanz in den Behälter erreicht werden, z. B. wenn dieser mit der Schicht in Kontakt tritt.

Von Vorteil ist es ferner, daß zumindest die obere, den Behälter bildende Schicht in etwa die gleiche Eigenschaft bzw. Dichte aufweist wie die als Membrane ausgebildete, den Behälter verschließende, Aromastoffe durchlassende Schicht und daß die beiden Schichten miteinander verschweißt sind.

Eine wesentliche, vorteilhafte Ausführungsform erreicht man dadurch, daß der Kleber zwischen der Barriereschicht bzw. der Außenfolie und dem Polypropylen eine geringere Haftfähigkeit zum Polypropylen hat als der Kleber zwischen dem Polypropylen und dem Polyäthylen.

Vorteilhaft ist es außerdem, daß die Dichte des Materials der Barriereschicht größer ist als die Dichte des Materials der oberen Schicht bzw. des Polypropylens und daß die Dichte des Materials der oberen Schicht bzw. des Polypropylens kleiner ist als die Dichte des Materials der unteren Schicht bzw. des Polyäthylens, wobei die Dichte der unteren Schicht der Membrane und der am Behälter anhaftenden, oberen Schicht gleich ist.

Ferner ist es vorteilhaft, daß die erste Schicht der Verbundfolie bzw. des Behälters eine Dicke zwischen 12 µm und 80 µm, die zweite Schicht eine Dicke zwischen 9 µm und 50 µm, die dritte Schicht eine Dicke zwischen 33 µm und 60 µm, die vierte Schicht eine Dicke zwischen 50 µm und 150 µm, die fünfte Schicht eine Dicke zwischen 50 µm und 100 µm und die sechste Schicht eine Dicke zwischen 250 µm und 500 µm aufweist.

Besonders vorteilhaft ist es außerdem, daß die erste Schicht der Verbundfolie bzw. des Behälters eine Dicke zwischen 20 µm und 30 µm, die zweite Schicht eine Dicke zwischen 12 µm und 18 µm, die dritte Schicht eine Dicke zwischen 28 µm und 38 µm, die vierte Schicht eine Dicke zwischen 70 µm und 80 µm, die fünfte Schicht eine Dicke zwischen 70 µm und 80 µm und die sechste Schicht eine Dicke zwischen 320 µm und 380 µm aufweist.

Sehr gute Ergebnisse wurden dadurch erzielt, daß die erste Schicht der Verbundfolie bzw. des Behälters eine Dicke zwischen 24 µm und 26 µm, die zweite Schicht eine Dicke zwischen 14 µm und 16 µm, die dritte Schicht eine Dicke zwischen 32 µm und 34 µm, die vierte Schicht eine Dicke zwischen 74 µm und 76 µm, die fünfte Schicht eine Dicke zwischen 74 µm und 76 µm und die sechste Schicht eine Dicke zwischen 345 µm und 355 µm aufweist.

Weitere Vorteile und Einzelheiten der Erfindung sind in den Patentansprüchen und in der Beschreibung erläutert und in den Figuren dargestellt, wobei bemerkt wird, daß alle Einzelmerkmale und alle Kombinationen von Einzelmerkmalen erfindungswesentlich sind. Es zeigt:
- Figur 1: eine Querschnittsdarstellung eines Behälters zur Aufnahme von aromatischen Substanzen, der mittels einer aus mehreren Schichten gebildeten Verbundfolie aromadicht verschließbar ist,
- Figur 2: eine Draufsicht mehrerer Behälter zur Aufnahme von aromatischen Substanzen, die über eine Folie miteinander verbunden sind,
- Figur 3: eine Perspektivische Darstellung eines Behälters mit teilweise abgezogener Außenfolie.

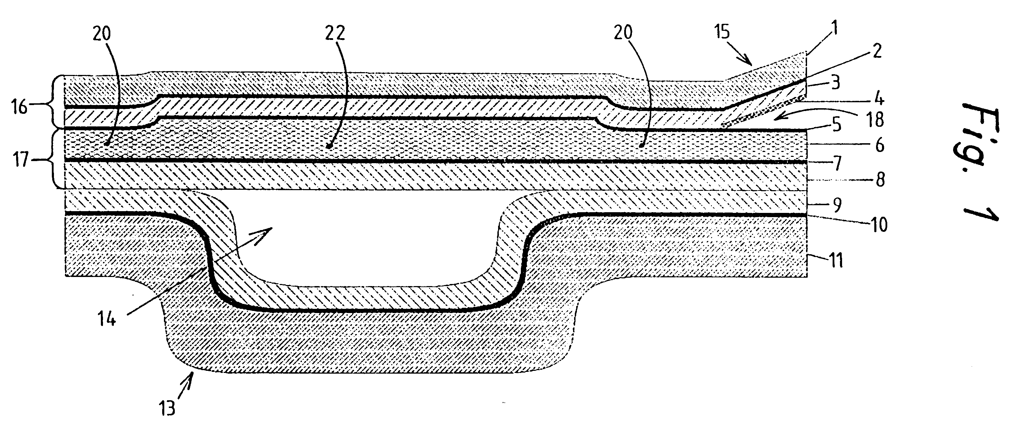

In der Zeichnung ist in Figur 1 ein Behälter 13 zur Aufnahme von aromatischen Substanzen 14 dargestellt, der mittels einer aus mehreren Schichten gebildeten Verbundfolie 15 aromadicht verschließbar ist. Im Ausführungsbeispiel gemäß Figur 1 besteht die Verbundfolie 15 einschließlich Behälter 13 aus insgesamt sechs Schichten 1, 3, 6, 8, 9, 11. Zählt man die dazwischen liegenden Kleber 2, 5, 7, 10 dazu, so besteht die Verbundfolie 15 einschließlich Behälter 13 aus zehn Schichten.

Eine Außenfolie 16 ist aus den beiden fest miteinander verbundenen Schichten 1, 3 gebildet, wobei die eine bzw. untere Schicht 3 als aromadichte Barriereschicht ausgebildet sein kann und die andere bzw. obere Schicht 1 aus einem elastischen bzw. biegsamen Material, insbesondere Polyamidwerkstoff oder beispielsweise auch Polypropylen, gebildet sein kann.

Die erste Schicht 1 stellt sicher, daß beim Abreißvorgang die als Verbundfolie ausgebildete Barriereschicht 16 nicht einreißt, so daß diese auf einfache Weise abgezogen werden kann, um die als Innenfolie ausgebildete Membranfolie 17 freizulegen und den Diffundiervorgang einer im Behälter 13 aufgenommenen, aromatischen Substanz 14 zu ermöglichen.

Im Gesamtverbund der Folie 15 ist die zweite Schicht 3 aus einem aromadichten Werkstoff gebildet.

Die von der Oberseite durchgezählt dritte Schicht 6 kann aus einem relativ weichen Polymerwerkstoff, vorzugsweise aus einem geschäumten Polypropylen, gebildet sein. Bei einer besonderen Bearbeitung und Einbindung in den Gesamtverbund der Folie 15 weist es, wie nachstehend noch beschrieben, sehr gute Dichteigenschaften auf und stellt eine vorteilhafte Verbindung zwischen den beiden Schichten 3 und 8 dar.

Die dritte Schicht bzw. das geschäumte Polypropylen 6 bildet zusammen mit der darunterliegenden und anhaftenden Schicht 8 die Innenfolie bzw. die Membranfolie 17. Die Schicht 8 kann aus Polyäthylen bestehen.

Die Dichte des Materials der unteren Schicht 3 der Barriereschicht 16 ist in vorteilhafter Weise größer als die Dichte des Materials der dritten Schicht bzw. des Polypropylens 6. Auf diese Weise ist eine einfache Trennung zwischen der Barriereschicht 16 und der Membranfolie 17 möglich, und es wird auch gewährleistet, daß beim Trennvorgang die aus Polypropylen gebildete dritte Schicht 6, die zwischen der Barriereschicht 16 und der Membranfolie 17 vorgesehen ist, nicht gespalten wird, sondern daß die Trennung entlang dem Kleber 5 verläuft.

Die Barriereschicht 3 kann aus einem Metallwerkstoff, insbesondere aus einem Al-Material, gebildet sein. Aus umwelttechnischen Gründen ist es aber zweckmäßig, wenn die Barriereschicht 3 aus einem nichtmetallischen Werkstoff gebildet ist. So kann die Barriereschicht 3 aus einem hydrolisierten Vinylazetat- und/oder Äthylen- und Copolymer-Material (EVOH und EVA) bestehen und mit der ersten Schicht 1 verbunden sein, die aus Polyamid oder aus Polypropylen bestehen kann, wodurch, wie bereits erwähnt, die Reißfestigkeit der Barriereschicht 3 wesentlich verbessert wird. Aufgrund seines Polyvinylalkoholgehalts besitzt EVOH sehr gute Barriere-Eigen schaften und dank seines Äthylengehalts sehr gute Hitze- und Wasserbeständigkeit.

Die aromadichte Barriereschicht 3 ist mit der anliegenden Schicht 1 ganzflächig fest verbunden. Die einzelnen Schichten 1, 3 und 6, 8 und 9, 11 können über je einen Kaschierkleber, z. B. einen Zweikomponentenkleber auf PU-Basis, einen Acrylkleber oder ein Koextrudierverfahren, ganzflächig fest miteinander verbunden werden.

Der Behälter 13 wird nach dem Einfüllen der aromatischen Substanz 14 mittels der Außenfolie bzw. der Barriereschicht 16 und einer aus zwei Schichten 6, 8 gebildeten Membranfolie 17 aromadicht verschlossen.

Die mit Bezug auf den Gesamtverbund der Folie 15 dritte zur Membrane 17 gehörende Schicht 6 ist aus einem geschäumten Polypropylen gebildet und mit einer vierten aus Polyäthylen bestehenden Schicht 8 über den Kaschierkleber 7 fest verbunden. Die beiden die Membrane 17 bildenden Schichten 6, 8 verschließen den Behälter auch dann, wenn die Außenfolie 16 abgezogen ist.

Die untere, zur Membrane 17 gehörende Schicht 8 ist mit der oberen bzw. fünften, auf dem Behälter 13 haftenden Schicht 9 verschweißt und somit unlösbar verbunden.

Der Behälter 13 besteht zur Aufnahme von aromatischen Substanzen 14 aus den beiden ganzflächig miteinander verbundenen Schichten 9 und 11, wobei die fünfte Schicht 9 des Behälters 13 aus Polyäthylen und die sechste bzw. außenliegende Schicht 11 aus einem Polymerwerkstoff, vorzugsweise Polyester oder Polypropylen, gebildet ist. Die beiden den Behälter 13 bildenden Schichten 9, 11 sind über einen Kaschierkleber 10 ganzflächig miteinander verbunden.

Die obere, den Behälter 13 bildende Schicht 9 weist in etwa die gleiche Eigenschaft bzw. Dichte auf wie die als Membrane ausgebildete, den Behälter 13 verschließende, Aromastoffe durchlassende, vierte Schicht 8. Hierdurch können die beiden Schichten 8, 9 ohne weiteres miteinander verschweißt und somit fest verbunden werden.

Ferner ist es sehr günstig, daß der Kleber 5 zwischen der Barriereschicht bzw. der Außenfolie 16 und dem Polypropylen 6 eine geringere Haftfähigkeit hat als der Kleber 7 zwischen dem Polypropylen 6 und dem Polyäthylen 8.

Die Dichte der zweiten bzw. oberen Schicht 3 ist so gewählt, daß beim Abziehen der Außenfolie 16 die gesamte Membranfolie 17 auf dem Behälter 13 haften bleibt, d. h. daß die Dichte der zweiten Schicht 3 größer ist als die Dichte der dritten Schicht 6.

Die aromadichte Barriereschicht 3 ist mit der anliegenden Schicht 1 ganzflächig fest verbunden. Die einzelnen auch nachfolgend näher erläuterten Schichten 1, 3 und 6, 8 und 9, 11 sind über je einen Kaschierkleber 2, 5, 7, 10 ganzflächig fest miteinander verbunden.

Die einzelnen Schichten 1, 3 und 6, 8 und 9, 11 können über je einen Zweikomponentenkleber, einen Acrylkleber oder ein Koextrudierverfahren ganzflächig fest miteinander verbunden werden.

Damit eine hohe Lagerstabilität der im Behälter 13 verschlossenen Substanz 14 gewährleistet ist, wird bei der Verbindung der Barriereschicht 16 mit der Membranfolie 17 der Randbereich der dritten Schicht bzw. des Polypropylens 6 und/oder der darunterliegenden Schichten 8, 9 verdichtet und über den Kaschierkleber fest verbunden. Der verdichtete Teil 20 der Schicht 6 ist in Figur 1 durch einen Absatz angedeutet. Er verhindert, daß die im Behälter 13 aufgenommene Substanz über den von dem Polpropylen 6 gebildeten Zwischenraum zwischen der Schicht 3 und der Schicht 8 nach außen diffundiert.

Ferner ist es möglich, die Stirnkanten der Schichten 6, 8, 9 zu versiegeln oder mit einer Barriereschicht zu versehen, so daß in geschlossenem Zustand des Behälters 13 kein Duftstoff über die Stirnfläche der Schichten 6, 8, 9 nach außen dringt. Ferner besteht die Möglichkeit, die obere Barriereschicht über die Stirnkanten der darunterliegenden Schichten 6, 8, 9 zu ziehen. Normalerweise wird aber schon durch den verdichteten Randbereich der Schicht 6 eine ausreichende Dichte geschaffen, so daß über die Schichten 6, 8, 9 kein Duftstoff nach außen diffundieren kann, solange die Schicht 3 nicht von der Schicht 6 abgezogen ist, insbesondere deshalb, weil diese Schichten sich in versiegeltem Zustand nicht vollsaugen können.

Der beiderseits des Polypropylens 6 vorgesehene Kleber 5, 7 kann so beschaffen sein, daß er zusätzlich das Diffundieren nach außen verhindert.

Wie aus Figur 1 hervorgeht, befindet sich im Randbereich der dritten Schicht 6 ein keilförmiger Spalt bzw. eine Trennstelle 18, so daß der im Randbereich liegende Teil der beiden Schichten 1, 3 eine Lasche bzw. Öse 19 bildet, mittels der die Barriereschicht 16 abgezogen werden kann. Im Spalt 18 kann die Unterseite der Schicht 3 mit einer Schutzschicht 4, insbesondere einem Flexodruck, einem Streifen aus Silikon oder rotem Silikonlack, versehen sein, so daß sich dieser Teil der Schicht 3 nicht mit dem darunterliegenden Teil der Schicht 6 verbindet und, insbesondere beim Abziehvorgang, die beiden oberen Schichten 1 und 3 mit dem Kaschierkleber 5 von der Oberfläche der dritten Schicht 6 bzw. des geschäumten Polypropylens abgezogen werden können, ohne dieses zu beschädigen. Nur im Randbereich bleibt der Kaschierkleber 5 auf der Oberfläche des Polypropylens 6 haften.

Sollen die Duftstoffe aktiviert werden, so muß lediglich die Außenfolie 16 mittels der Lasche 19 abgezogen werden, wobei die Trennlinie auf der Ebene des Klebers 5 oder entlang der Unterseite des Klebers 5 verläuft. Nun können die Duftstoffe durch die Schichten 8 und 6 hindurchtreten. Da ein mittlerer, oberhalb der Vertiefung im Behälter 13 liegender Teil 22 der Schicht 6 nicht verdichtet wurde, können die Duftstoffe mit einer konstanten Diffundierrate durch die beiden Schichten 6, 8 austreten.

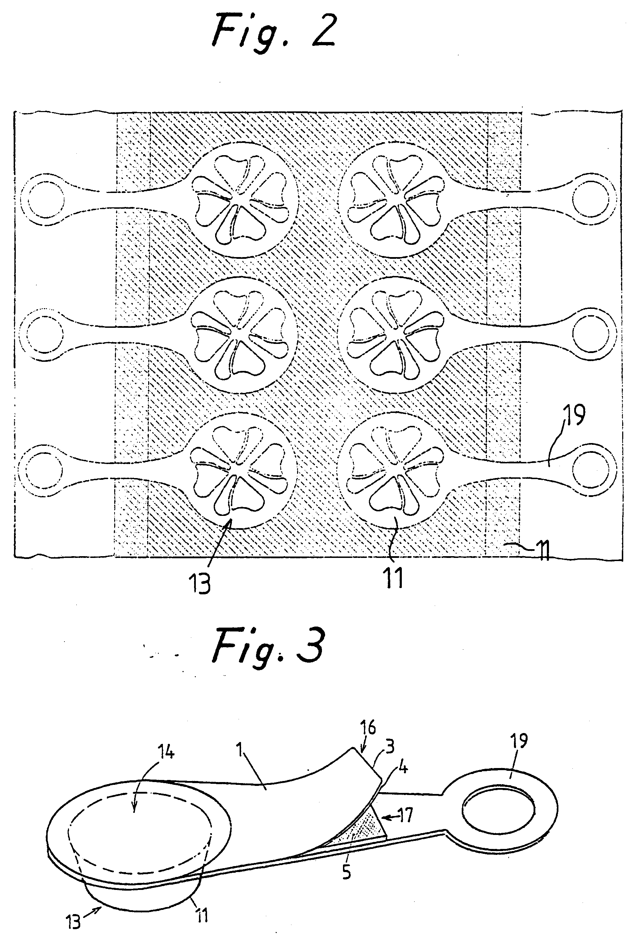

In Figur 2 sind mehrere Behälter 13 beim Herstellungsverfahren über eine Folie, z. B. die untere Folie bzw. die Schicht 11, miteinander verbunden, damit sie als Mehrfachpackung gehandelt werden können. Diese Behälter 13 können herausgestanzt oder mittels einer in der Zeichnung nicht dargestellten Perforation aus der Folie 11 herausgelöst werden.

Ferner können die beiden unteren Schichten 9, 11 seitlich des Behälters so verlängert sein, daß der überstehende Teil als Öse 19 ausgebildet sein kann, mittels der der Behälter 13 z. B. an einem Haken befestigt werden kann.

### Bezugszeichenliste

- 1: erste Schicht, Pa Polyamid, Stützschicht
- 2: Kaschierkleber
- 3: zweite Schicht, Barriereschicht
besteht aus Metall, insbesondere Al
- 4: Schutzschicht, Flexodruck, Streifen aus Silikon, rot
- 5: Kaschierkleber, Bindemittel
- 6: dritte Schicht, geschäumtes Polypropylen, PP=Polymer
- 7: Kaschierkleber, Bindemittel
- 8: vierte Schicht, Polyäthylen
- 9: fünfte Schicht, Polyäthylen
- 10: Kaschierkleber
- 11: sechste Schicht, Polyester, Polypropylen
- 13: Behälter
- 14: aromatische Substanz
- 15: Verbundfolie
- 16: Außenfolie, Barriereschicht, 1. und 2. Schicht (1, 3) EVOH und EVA-Material entspricht hydrolisiertem Vinylazetat, Äthylen und Copolymer
und kann ebenfalls als Barriereschicht eingesetzt werden
- 17: Innenfolie, Membranfolie, 3. und 4. Schicht (6, 8)
- 18: Trennstelle
- 19: Lasche, Öse
- 20: verdichteter Teil
- 22: unverdichteter Teil

## Patentansprüche

1. Behälter (13) zur Aufnahme von aromatischen Substanzen (14), der mittels einer aus mehreren Schichten gebildeten Verbundfolie (15) aromadicht verschließbar ist, wobei die Verbundfolie (15) auf einer Behälteroberseite aus einer aromadichten, mindestens zwei Schichten (1, 3) aufweisenden Außenfolie (16) und einer darunterliegenden, aromadurchlässigen, aus zwei Schichten (6, 8) bestehenden Innenfolie (17) gebildet ist, wobei die aromadichte Außenfolie (16) mit der darunterliegenden, aus einem Polymerwerkstoff gebildeten, permeablen Zwischenschicht (6) über einen Kleber (5) verbunden ist, **dadurch gekennzeichnet,** daß der Kleber eine geringere Haftfähigkeit zu der Zwischenschicht (6) aufweist als der zweite Kleber (7) zwischen der Zwischenschicht (6) und der nachfolgenden vierten, mit dem Behälter (13) verbundenen Schicht (8), die aus Polyäthylen besteht, und daß mit Bezug auf den Gesamtverbund der Folie (15) die dritte bzw. die zur Innenfolie gehörende Zwischenschicht (6) aus einem geschäumten Polypropylen gebildet ist.

2. Behälter nach Anspruch 1, **dadurch gekennzeichnet,** daß die Dichte der dritten bzw. oberen Schicht (6) so gewählt ist, daß die aus den beiden Schichten (6, 8) gebildete Membranfolie (17) beim Abziehen der Außenfolie (16) auf dem Behälter (13) haften bleibt.

3. Behälter nach Anspruch 1 oder 2, **dadurch gekennzeichnet**, daß die dritte Schicht bzw. das geschäumte Polypropylen (6) im Randbereich fest mit dem darunterliegenden Polyäthylen (8) verbunden ist und das geschäumte Polypropylen (6) und/oder die darunterliegenden Schichten (8, 9) im Randbereich der festen Verbindung verdichtet sind.

4. Behälter nach Anspruch 3, **dadurch gekennzeichnet,** daß das geschäumte Polypropylen (6) im Bereich der festen Verbindung durch Wärmebehandlung verdichtet ist.

5. Behälter nach einem oder mehreren der vorhergehenden Ansprüche, **dadurch gekennzeichnet,** daß das geschäumte Polypropylen (6) über den Kleber (7) ganzflächig mit dem darunterliegenden Polyäthylen (8) fest verbunden und nur im Randbereich und/oder insbesondere im Bereich der Klebe- oder Siegelstelle verdichtet ist.

6. Behälter nach einem oder mehreren der vorhergehenden Ansprüche, **dadurch gekennzeichnet,** daß die Außenfolie (16) aus zwei fest miteinander verbundenen Schichten (1, 3) gebildet ist, wobei die eine Schicht (3) als aromadichte Barriereschicht und die andere Schicht (1) aus einem biegsamen bzw. elastischen Material, insbesondere Polyamidwerkstoff, gebildet ist.

7. Behälter nach einem oder mehreren der vorhergehenden Ansprüche, **dadurch gekennzeichnet,** daß die Barriereschicht (3) aus einem Metallwerkstoff bzw. aus Al-Material gebildet ist.

8. Behälter nach einem oder mehreren der vorhergehenden Ansprüche, **dadurch gekennzeichnet,** daß die aromadichte Barriereschicht (3) mit der anliegenden, aus Polyamid gebildeten Schicht (1) ganzflächig fest verbunden ist.

9. Behälter nach einem oder mehreren der vorhergehenden Ansprüche, **dadurch gekennzeichnet,** daß der Behälter (13) zur Aufnahme von aromatischen Substanzen (14) aus zwei ganzflächig miteinander verbundenen Schichten (9, 11) gebildet ist, wobei die Schicht (9) des Behälters (13) aus Polyäthylen und die außenliegende Schicht (11) aus Polyester oder Polypropylen gebildet ist.

10. Behälter nach einem oder mehreren der vorhergehenden Ansprüche, **dadurch gekennzeichnet,** daß die einzelnen Schichten (1, 3 und 6, 8 und 9, 11) über je einen Kaschierkleber (2, 5, 7, 10) ganzflächig fest miteinander verbunden sind.

11. Behälter nach einem oder mehreren der vorhergehenden Ansprüche, **dadurch gekennzeichnet,** daß die Außenfolie (16) mit der Innenfolie bzw. Membranfolie (17) im Randbereich verschweißt oder über den Kaschierkleber (5) verbunden ist.

12. Behälter nach einem oder mehreren der vorhergehenden Ansprüche, **dadurch gekennzeichnet,** daß die Außenfolie (16) mit der Innenfolie bzw. Membranfolie (17) ganzflächig verschweißt oder über den Kaschierkleber (5) verbunden ist, wobei im Randbereich eine Trennstelle (18) für eine Öffnungslasche (19) vorgesehen ist, die derart ausgebildet ist, daß mit ihr die Außenfolie (16) von der Innenfolie (17) abgezogen und der Verbund zwischen Innenfolie (17) und Außenfolie (16) im Bereich des Kaschierklebers (5) ganzflächig von der Polypropylen-Schicht (6) abgehoben werden kann.

13. Behälter nach einem oder mehreren der vorhergehenden Ansprüche, **dadurch gekennzeichnet,** daß der Randbereich der Unterseite der Außenfolie (16) oder der zweiten Schicht (3) mit einer Schutzschicht (4), insbesondere einem Lackstreifen, zur Verhinderung einer Verbindung der Unterseite der zweiten Schicht (3) mit der Oberseite der dritten Schicht (6) versehen ist.

14. Behälter nach einem oder mehreren der vorhergehenden Ansprüche, **dadurch gekennzeichnet,** daß die zweite Schicht (3) der Außenfolie (16) aus einem metallfreien Copolymer-Werkstoff, nämlich EVA und EVOH (EVOH und EVA), besteht und mit der ersten Schicht (1) verbunden ist, die aus einem biegsamen Material, insbesondere aus Polyamid oder Polypropylen, besteht.

15. Behälter nach einem oder mehreren der vorhergehenden Ansprüche, **dadurch gekennzeichnet,** daß die einzelnen Schichten (1, 3 und 6, 8 und 9, 11) über je einen Zweikomponentenkleber auf PU-Basis, einen Acrylkleber oder ein Koextrudierverfahren ganzflächig miteinander verbunden sind.

16. Verfahren zur Herstellung einer Folie nach Anspruch 1, **dadurch gekennzeichnet,** daß durch Wärmebehandlung des Polypropylenmaterials bzw. der Membranfolie (17) bzw. der Polyäthylenfolie (8) oder durch Eingabe der Substanz in den Behälter und Kontaktaufnahme mit der Schicht die beiden Folien derart transparent gemacht werden, daß die im Behälter aufgenommene Substanz durch die beiden Schichten (6, 8) sichtbar ist, wenn die Außenfolie abgezogen wurde.

17. Behälter nach einem oder mehreren der vorhergehenden Ansprüche, **dadurch gekennzeichnet,** daß zumindest die obere, den Behälter bildende Schicht (9) in etwa die gleiche Eigenschaft bzw. Dichte aufweist wie die als Membrane ausgebildete, den Behälter (13) verschließende, Aromastoffe durchlassende Schicht (8) und daß die beiden Schichten (8, 9) miteinander verschweißt sind.

18. Behälter nach einem oder mehreren der vorhergehenden Ansprüche, **dadurch gekennzeichnet,** daß die Dichte des Materials der Barriereschicht (3) größer ist als die Dichte des Materials der oberen Schicht bzw. des Polypropylens (6) und daß die Dichte des Materials der oberen Schicht bzw. des Polypropylens (6) kleiner ist als die Dichte des Materials der unteren Schicht bzw. des Polyäthylens (8), wobei die Dichte der unteren Schicht (8) der Membrane und der am Behälter (13) anhaftenden, oberen Schicht (9) gleich ist.

19. Behälter nach einem oder mehreren der vorhergehenden Ansprüche, **dadurch gekennzeichnet,** daß die erste Schicht (1) der Verbundfolie bzw. des Behälters (13) eine Dicke zwischen 12 µm und 80 µm, die zweite Schicht (3) eine Dicke zwischen 9 µm und 50 µm, die dritte Schicht (6) eine Dicke zwischen 33 µm und 60 µm, die vierte Schicht (8) eine Dicke zwischen 50 µm und 150 µm, die fünfte Schicht (9) eine Dicke zwischen 50 µm und 100 µm und die sechste Schicht (11) eine Dicke zwischen 250 µm und 500 µm aufweist.

20. Behälter nach einem oder mehreren der vorhergehenden Ansprüche, **dadurch gekennzeichnet,** daß die erste Schicht (1) der Verbundfolie bzw. des Behälters (13) eine Dicke zwischen 20 µm und 30 µm, die zweite Schicht (3) eine Dicke zwischen 12 µm und 18 µm, die dritte Schicht (6) eine Dicke zwischen 28 µm und 38 µm, die vierte Schicht (8) eine Dicke zwischen 70 µm und 80 µm, die fünfte Schicht (9) eine Dicke zwischen 70 µm und 80 µm und die sechste Schicht (11) eine Dicke zwischen 320 µm und 380 µm aufweist.

21. Behälter nach einem oder mehreren der vorhergehenden Ansprüche, **dadurch gekennzeichnet,** daß die erste Schicht (1) der Verbundfolie bzw. des Behälters (13) eine Dicke zwischen 24 µm und 26 µm, die zweite Schicht (3) eine Dicke zwischen 14 µm und 16 µm, die dritte Schicht (6) eine Dicke zwischen 32 µm und 34 µm, die vierte Schicht (8) eine Dicke zwischen 74 µm und 76 µm, die fünfte Schicht (9) eine Dicke zwischen 74 µm und 76 µm und die sechste Schicht (11) eine Dicke zwischen 345 µm und 355 µm aufweist.

## Claims

1. Container (13) to hold aromatic substances (14) which can be sealed aroma-impermeable by means of a composite film (15) consisting of several layers, whereby the composite film (15) on the top of the container is made up of an aroma-impermeable outer film (16) having at least two layers (1, 3) and, below it, of an aroma-permeable inner film (17) consisting of two layers (6, 8), whereby the aroma-impermeable outer film (16) is bonded to the permeable intermediate layer (6) below it consisting of a polymer material by means of an adhesive (5), **characterized in that** the adhesive adheres less strongly to the intermediate layer (6) than the second adhesive (7) between the intermediate layer (6) and the subsequent fourth layer (8) which consists of polyethylene and is bonded to the container (13), and in that the layer which is the third with respect to the entire composite film arrangement (15), that is to say, the layer (6) belonging to the inner film, is made of foamed polypropylene.

2. Container according to Claim 1, **characterized in that** the density of the third or top layer (6) is selected in such a way that the membrane film (17) made up of the two layers (6, 8) stays on the container (13) when the outer film (16) is pulled off.

3. Container according to Claim 1 or 2, **characterized in that** the third layer or foamed polypropylene (6) is permanently bonded to the polyethylene (8) below it, in the edge area, and the foamed polypropylene (6) and/or the layers (8, 9) below it are crimped in the edge area which has been permanently bonded.

4. Container according to Claim 3, **characterized in that** the foamed polypropylene (6) is crimped in the area which has been permanently bonded by means of a heat treatment.

5. Container according to one or more of the preceding Claims, **characterized in that** the foamed polypropylene (6) is permanently bonded to the polyethylene (8) below it over the entire surface by means of the adhesive (7) and is only crimped in the edge area and/or especially in the area of the adhesion or sealing site.

6. Container according to one or more of the preceding Claims, **characterized in that** the outer film (16) consists of two layers (1, 3) permanently bonded to each other, whereby one of the layers (3) is formed as an aroma-impermeable barrier layer and the other layer (1) is made of a flexible or elastic material, especially of a polyamide material.

7. Container according to one or more of the preceding Claims, **characterized in that** the barrier layer (3) is made of a metal, especially of an aluminum material.

8. Container according to one or more of the preceding claims, **characterized in that** the aroma-impermeable barrier layer (3) is permanently bonded to the adjacent layer (1) made of polyamide over the entire surface.

9. Container according to one or more of the preceding claims, **characterized in that** the container (13) to hold aromatic substances (14) is made of two layers (9, 11) bonded to each other over the entire surface, whereby the layer (9) of the container (13) is made of polyethylene and the outer layer (11) is made of polyester or polypropylene.

10. Container according to one or more of the preceding claims, **characterized in that** the individual layers (1, 3 and 6, 8 and 9, 11) are each permanently bonded to each other over the entire surface by means of an adhesive (2, 5, 7, 10).

11. Container according to one or more of the preceding claims, **characterized in that** the outer film (16) is fused with the inner film or membrane film (17) in the edge area or else is bonded by means of the adhesive (5).

12. Container according to one or more of the preceding claims, **characterized in that** the outer film (16) is fused over the entire surface with the inner film or membrane film (17) or else it is bonded by means of the adhesive (5), whereby in the edge area, there is a separation line (18) for an opening tab (19) which is designed in such a way that it can be used to pull the outer film (16) away from the inner film (17) and the bond between the inner film (17) and outer film (16) in the area of the adhesive (5) can be separated over the entire surface from the polypropylene layer (6).

13. Container according to one or more of the preceding claims, **characterized in that** the edge area of the underside of the outer film (16) or of the second layer (3) has a protective layer (4), especially a strip of coating, in order to prevent a bonding of the underside of the second layer with the top of the third layer (6).

14. Container according to one or more of the preceding claims, **characterized in that** the second layer (3) of the outer film (16) consists of a non-metallic material, namely, EVOH and EVA and is bonded to the first layer (1), which consists of a flexible material, especially one made of polyamide or polypropylene.

15. Container according to one or more of the preceding claims, **characterized in that** the individual layers (1, 3 and 6, 8 and 9, 11) are permanently bonded to each other over the entire surface by employing a two-component adhesive on the basis of PU, an acrylic adhesive or a coextrusion process.

16. Process for the production of a film according to Claim 1, **characterized in that** the two films are rendered transparent through heat treatment of the polypropylene material or membrane film (17) or polyethylene film (8), or else in that the substance is placed into the container and put into contact with the layer, so that the substance held in the container is visible through the two layers (6, 8) when the outer film has been pulled off.

17. Container according to one or more of the preceding claims, **characterized in that** at least the top layer (9) forming the container has approximately the same properties or density as the aroma-permeable layer (8) which functions as a membrane and also seals the container (13) and allows aromatic substances to pass through, and in that the two layers (8, 9) are fused together.

18. Container according to one or more of the preceding claims, **characterized in that** the density of the material of the barrier layer (3) is greater than the density of the material of the top layer or polypropylene (6), and in that the density of the material of the top layer or polypropylene (6) is less than the density of the material of the bottom layer or polyethylene (8), whereby the density of the bottom layer (8) of the membrane and of the upper layer (9) adhering to the container (13) is the same.

19. Container according to one or more of the preceding claims, **characterized in that** the first layer (1) of the composite film or of the container (13) has a thickness between 12 µm and 80 µm, the second layer (3) has a thickness between 9 µm and 50 µm, the third layer (6) has a thickness between 33 µm and 60 µm, the fourth layer (8) has a thickness between 50 µm and 150 µm, the fifth layer (9) has a thickness between 50 µm and 100 µm and the sixth layer (11) has a thickness between 250 µm and 500µm.

20. Container according to one or more of the preceding claims, **characterized in that** the first layer (1) of the composite film or of the container (13) has a thickness between 20 µm and 30 µm, the second layer (3) has a thickness between 12 µm and 18 µm, the third layer (6) has a thickness between 28 µm and 38µm, the fourth layer (8) has a thickness between 70 µm and 80 µm, the fifth layer (9) has a thickness between 70 µm and 80 µm and the sixth layer (11) has a thickness between 320 µm and 380 µm.

21. Container according to one or more of the preceding claims, **characterized in that** the first layer (1) of the composite film or of the container (13) has a thickness between 24 µm and 26 µm, the second layer (3) has a thickness between 14 µm and 16 µm, the third layer (6) has a thickness between 32 µm and 34 µm, the fourth layer (8) has a thickness between 74 µm and 76 µm, the fifth layer (9) has a thickness between 74 µm and 76 µm and the sixth layer (11) has a thickness between 345 µm and 355 µm.

## Revendications

1. Récipient (13) pour substances aromatiques (14), pouvant être fermé par un film composite (15) imperméable aux arômes composé de plusieurs couches, le film composite (15) sur un côté supérieur du récipient étant constitué par un film extérieur (16) imperméable aux arômes qui comprend au moins deux couches (1, 3) et par un film intérieur (17) disposé au-dessous perméable aux arômes qui comprend deux couches (6, 8), le film extérieur (16) imperméable aux arômes étant rendu solidaire de la couche intermédiaire (6) perméable aux arômes constituée d'un matériau polymère par un adhésif (5), **caractérisé en ce que** le pouvoir adhésif de la colle est moins fort côté couche intermédiaire (6) que celui du deuxième adhésif (7) entre la couche intermédiaire (6) et la quatrième couche suivante (8) solidaire du récipient (13) constituée de polyéthylène, et que, rapporté à l'ensemble du film composite (15), la troisième couche, à savoir la couche intermédiaire (6) faisant partie du film intérieur consiste en polypropylène moussé.

2. Récipient selon la revendication 1, **caractérisé en ce que** le choix retenu pour la densité de la troisième couche, à savoir la couche supérieure (6) soit tel que le film membranaire (17) constitué par les deux couches (6, 8) adhère toujours bien au récipient (13) lors du retrait du film extérieur (16).

3. Récipient selon l'une des revendications 1 ou 2, **caractérisé en ce que** la troisième couche, à savoir la couche en polypropylène moussé (6) est fermement solidaire dans la zone périphérique du polyéthylène (8) disposé en dessous, et que le polypropylène moussé (6) et/ou les couches du dessous (8, 9) sont comprimés dans la zone périphérique de la jonction solide.

4. Récipient selon la revendication 3, **caractérisé en ce que** le polypropylène moussé (6) dans la zone de la jonction solide est comprimé par traitement thermique.

5. Récipient selon l'une ou plusieurs des revendications ci-avant, **caractérisé en ce que** le polypropylène expansé (6) est rendu fermement solidaire sur toute sa surface du polyéthylène (8) disposé au-dessous par l'adhésif (7) et est densifié uniquement dans la zone périphérique et/ou notamment aux endroits scellés ou collés.

6. Récipient selon l'une ou plusieurs des revendications ci-avant, **caractérisé en ce que** le film extérieur (16) est constitué par deux couches (1, 3) solidaires l'une de l'autre, l'une de ces couches (3) faisant barrière contre la perte d'arômes, et l'autre couche (1) consistant en un matériau souple-élastique, notamment un matériau polyamide.

7. Récipient selon l'une ou plusieurs des revendications ci-avant, **caractérisé en ce que** la couche barrière contre la perte d'arômes (3) consiste en un matériau métallique, ou d'aluminium.

8. Récipient selon l'une ou plusieurs des revendications ci-avant, **caractérisé en ce que** la couche barrière contre la perte d'arômes (3) est fermement solidaire sur toute sa surface de la couche adjacente (1) qui consiste en polyamide.

9. Récipient selon l'une ou plusieurs des revendications ci-avant, **caractérisé en ce que** le récipient (13) pour substances aromatiques (14) est constitué par deux couches (9, 11) fermement solidaires l'une de l'autre sur toute leur surface, la couche (9) du récipient (13) étant en polyéthylène, et la couche (11) disposée à l'extérieur étant en polyester ou en polypropylène.

10. Récipient selon l'une ou plusieurs des revendications ci-avant, **caractérisé en ce que** chacune des couches (1, 3 et 6, 8 et 9, 11) sont rendues fermement solidaires les unes des autres sur toute leur surface par un adhésif de doublage (2, 5, 7, 10).

11. Récipient selon l'une ou plusieurs des revendications ci-avant, **caractérisé en ce que** le film extérieur (16) est rendu solidaire du film intérieur, à savoir avec le film membranaire (17) dans la zone périphérique par soudage ou par un adhésif de doublage (5).

12. Récipient selon l'une ou plusieurs des revendications ci-avant, **caractérisé en ce que** le film extérieur (16) est rendu solidaire du film membranaire (17) sur toute sa surface par soudage ou par un adhésif de doublage (5), un rabat repliable (18) étant prévu dans la zone périphérique pour une languette d'ouverture (19) conçue de façon à entraîner avec elle le film extérieur (16) et à le détacher du film intérieur (17), et que le complexe entre le film intérieur (17) et le film extérieur (16) peut être détaché sur toute sa largeur de la couche de polypropylène (6) dans la zone de l'adhésif de doublage (5).

13. Récipient selon l'une ou plusieurs des revendications ci-avant, **caractérisé en ce que** la zone périphérique de la face inférieure du film extérieur (16) ou de la seconde couche (3) est enduite d'une couche de protection, notamment d'une laque, afin d'empêcher le scellage de la face inférieure de la deuxième couche (3) et de la face supérieure de la troisième couche (6).

14. Récipient selon l'une ou plusieurs des revendications ci-avant, **caractérisé en ce que** la seconde couche (3) du film extérieur (16) consiste en un matériau copolymère exempt de métaux, à savoir EVA et EVOH, et est rendu solidaire de la première couche (1) qui est constituée par un matériau souple, notamment le polyamide ou le polypropylène.

15. Récipient selon l'une ou plusieurs des revendications ci-avant, **caractérisé en ce que** les couches (1, 3 et 6, 8 et 9, 11) sont chacunes rendues solidaires les unes des autres sur toute leur surface par une colle à deux composants à base de PU, une colle acrylique, ou un procédé de coextrusion.

16. Procédé pour la fabrication d'un film selon la revendication 1, **caractérisé en ce que** en ayant recours à un traitement thermique du matériau de polypropylène ou du film membranaire (17) ou du film de polyéthylène (8), ou en incorporant la substance dans le récipient, par la mise en contact avec la couche, les deux films deviennent si transparents que la substance incorporée dans le récipient est visible à travers les deux couches (6, 8) après le retrait du film extérieur.

17. Récipient selon l'une ou plusieurs des revendications ci-avant, **caractérisé en ce que** au moins la couche (9) qui constitue la couche supérieure du récipient possède grosso modo les mêmes caractéristiques en termes de densité que celles de la couche (8) non barrière aux arômes faisant office de membrane et qui referme le récipient (13), et que les deux couches (8, 9) sont soudées l'une à l'autre.

18. Récipient selon l'une ou plusieurs des revendications ci-avant, **caractérisé en ce que** la densité du matériau de la couche barrière contre la perte d'arômes (3) est plus élevée que la densité du matériau de la couche supérieure, ou du polypropylène (6), et que la densité du matériau de la couche supérieure (6), ou du polypropylène (6) est moins élevée que la densité du matériau de la couche inférieure, ou du polyéthylène (8), la densité de la couche inférieure (8) de la membrane étant égale à celle de la couche supérieure (9) qui adhère au récipient (13).

19. Récipient selon l'une ou plusieurs des revendications ci-avant, **caractérisé en ce que** l'épaisseur de la première couche (1) du film composite ou du récipient (13) est comprise entre 12 et 80 µm, celle de la deuxième couche (3) entre 9 et 50 µm, celle de la troisième couche (6) entre 33 et 60 µm, celle de la quatrième couche (8) entre 50 et 150 µm, celle de la cinquième couche (9) entre 50 et 100 µm, et celle de la sixième couche (11) entre 250 et 500 µm.

20. Récipient selon l'une ou plusieurs des revendications ci-avant, **caractérisé en ce que** l'épaisseur de la première couche (1) du film composite ou du récipient (13) est comprise entre 20 et 30 µm, celle de la deuxième couche (3) entre 12 et 18 µm, celle de la troisième couche (6) entre 28 et 38 µm, celle de la quatrième couche (8) entre 70 et 80 µm, celle de la cinquième couche (9) entre 70 et 80 µm, et celle de la sixième couche (11) entre 320 et 380 µm.

21. Récipient selon l'une ou plusieurs des revendications ci-avant, **caractérisé en ce que** l'épaisseur de la première couche (1) du film composite ou du récipient (13) est comprise entre 24 et 26 µm, celle de la deuxième couche (3) entre 14 et 16 µm, celle de la troisième couche (6) entre 32 et 34 µms, celle de la quatrième couche (8) entre 74 et 76 µm, celle de la cinquième couche (9) entre 74 et 76 µm, et celle de la sixième couche (11) entre 345 et 355 µm.
